(19) Europäisches Patentamt
European Patent Office
Office européen des brevets

(11) **EP 2 159 222 A1**

(12) **EUROPÄISCHE PATENTANMELDUNG**

(43) Veröffentlichungstag:
**03.03.2010 Patentblatt 2010/09**

(51) Int Cl.:
***C07D 209/02*** *(2006.01)* ***C08K 5/00*** *(2006.01)*
***C08K 5/3417*** *(2006.01)* ***H04R 19/01*** *(2006.01)*

(21) Anmeldenummer: **08015097.2**

(22) Anmeldetag: **27.08.2008**

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(71) Anmelder: **Bayer MaterialScience AG**
**51368 Leverkusen (DE)**

(72) Erfinder:
- **Jenninger, Werner Dr.**
  **50674 Köln (DE)**
- **Wagner, Joachim Dr.**
  **51061 Köln (DE)**
- **Schmidt, Hans-Werner Prof. Dr.**
  **95444 Bayreuth (DE)**
- **Erhard, Dominik**
  **95444 Bayreuth (DE)**

(54) **Additive zur Verbesserung des Elektretverhaltens von Polycarbonaten**

(57) Die vorliegende Erfindung betrifft eine Zusammensetzung, welche mindestens ein Polycarbonat und mindestens eine Verbindung der allgemeinen Formel (I)

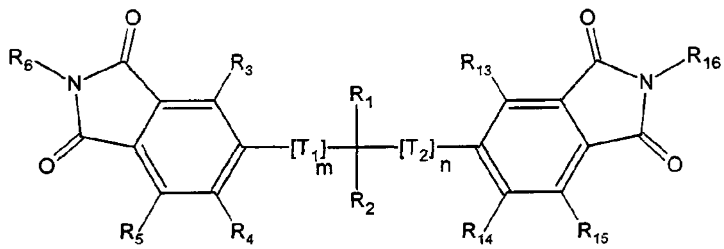

umfasst, ein diese Zusammensetzung umfassendes Polymerelement, ein Verfahren zur Herstellung eines solchen Polymerelement, ein aufgeladenes Polymerelement umfassendes Elektretelement, ein Verfahren zur Herstellung eines solchen Elektretelements, einen elektromechanischen Wandler auf der Basis eines solchen Polymer- oder Elektretelements, die Verwendung einer Verbindung der allgemeinen Formel (I) als Additiv zur Verbesserung der Elektreteigenschaften eines Polycarbonats sowie die Verwendung eines solchen Polymer- oder Elektretelements.

EP 2 159 222 A1

## Beschreibung

Ein Elektret ist ein Dielektrikum, das eine quasi-permanente elektrische Ladung und/oder Polarisierung trägt. Solche Elektrete finden vielfach Verwendung als akustische Membranen, beispielsweise in Mikrofonen, Sensoren und/oder Aktuatoren.

Weiterhin lassen sich Polymerelektrete als selbsthaftende, rückstandslos ablösbare und wiederverwendbare Filme, beispielsweise im Designbereich, in der Plakatwerbung oder bei Informationsschildern verwenden.

Insbesondere werden Polymerelektrete in elektromechanischen, beispielsweise piezoelektrischen, Anordnungen, beispielsweise in Lautsprechern, Schwingungswandlern, pyroelektrischen Detektoren und Kondensatoren, eingesetzt. Als elektromechanisch beziehungsweise piezoelektrisch bezeichnet man dabei die Fähigkeit, ein elektrisches Potential aufgrund einer ausgeübten mechanischen Belastung auszubilden. Eine elektromechanische, beispielsweise piezoelektrische, Anordnung kann unter anderem durch eine, insbesondere über die Dicke, heterogene Struktur realisiert werden. Beispielsweise sind Mehrschichtanordnungen aus übereinander gestapelten Polymerschäumen und kompakten Polymerfilmen bekannt, in denen die Polymerfilme als Elektret dienen und Ladungen tragen, welche bei mechanischer Beanspruchung der Anordnung zu einer elektrischen Reaktion führen.

In heutigen, kommerziell erhältlichen Elektretfilmen, wie sie beispielsweise in Mikrofonen zum Einsatz kommen, wird fast ausschließlich Polytetrafluoroethylen (PTFE) beziehungsweise Polytetrafluoroethylenpropylencopolymer (FEP) als Elektretmaterial verwendet. Der Vorteil dieser fluorierten Polymere ist, dass sie ein exzellentes Ladungsspeicherverhalten zeigen. Allerdings sind diese Polymere sehr kostspielig und zum anderen, vor allem im Fall von Polytetrafluoroethylen, schwierig zu verarbeiten.

Mittlerweile ist es auch gelungen, die Elektreteigenschaften des günstigen und leicht verarbeitbaren Massenkunststoff Polypropylen (PP) durch gezielte Additivierung signifikant zu verbessern. Beispielsweise zeigen Mohmeyer et al. (Macromolecules, 2006, 39, Seiten 5760 bis 5767), dass bereits geringe Konzentrationen substituierter 1,4-Phenylen-bisamide das Elektretverhalten von Corona-aufgeladenen Polypropylenfilmen signifikant erhöhen. Ein großer Nachteil von Polypropylen ist jedoch, dass dieses nicht dauerhaft oberhalb von 70°C als Elektret eingesetzt werden kann, da ab dieser Temperatur ein Ladungsabfall auftritt, der zum vollkommenen Verlust der Elektreteigenschaften von Polypropylen führen kann.

Die EP 0 845 058 B1 beschreibt zahlreiche unterschiedliche fluorhaltige Verbindungen, welche die Elektreteigenschaften von zahlreichen unterschiedlichen Polymeren und daraus ausgebildeten Netzen steigern sollen. In den Beispielen der EP 0 845 058 B1 werden Eigenschaften - wie das Basisgewicht, der effektive Faserdurchmesser, die Dicke, die Druckentspannung und der Qualitätsfaktor - von mit Verbindungen gemäß der EP 0 845 058 B1 additivierten Polypropylen-Netzen mit unaddivierten Polypropylen-Netzen verglichen.

Herkömmlicherweise sind jedoch zur Verbesserung des Elektretverhaltens eines speziellen Polymers aufgefundene Additive nicht zur Verbesserung des Elektretverhaltens anderer Polymere geeignet. So sind beispielsweise die von Mohmeyer et al. für Polypropylen aufgefundenen Additive nicht zur Verbesserung des Elektretverhaltens von Polycarbonaten geeignet, sondern führen sogar zu einem beschleunigten Ladungsabfall.

Die US 4,196,277 und US 5,116,939 beschreiben Additive zur Erhöhung der Temperaturstabilität beziehungsweise zur Verbesserung der Schmelzflusseigenschaften und/oder Verarbeitbarkeit von Polybenzimidazolen, Polypyrronen und/oder Polyimiden. Aus der Fähigkeit eines Additivs die Temperaturstabilität, die Schmelzflusseigenschaften und/oder die Verarbeitbarkeit eines Polymers zu verbessern, kann jedoch nicht abgeleitet werden, ob und wie ein derartiges Additiv das Elektretverhalten des Polymers beeinflusst.

Aufgabe der vorliegenden Erfindung ist es daher, eine Polymerzusammensetzung, ein Polymerelement beziehungsweise ein Polymerelektret zur Verfügung zu stellen, welche/s sowohl positive als auch negative Ladung, insbesondere quasi-permanent, tragen kann, mittels herkömmlichen Methoden leicht verarbeitet werden kann, bei Temperaturen von über 100°C noch gute Ladungsspeichereigenschaften aufweist und vorzugsweise, insbesondere für optische Anwendungen, transparent ist.

Diese Aufgabe wird gelöst durch eine erfindungsgemäße Zusammensetzung, umfassend

- mindestens ein Polycarbonat und
- mindestens eine Verbindung der allgemeinen Formel (I)

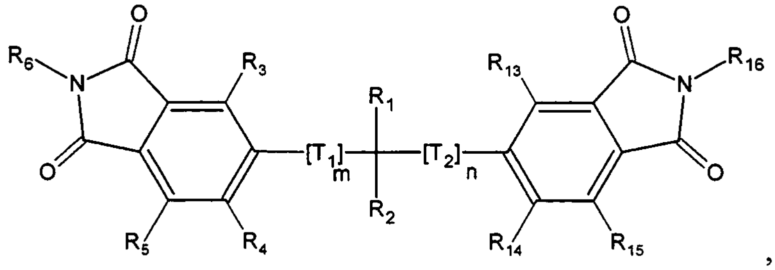

wobei

| | |
|---|---|
| $R_1$, $R_2$, $R_3$, $R_4$, $R_5$, $R_6$, $R_{13}$, $R_{14}$, $R_{15}$, $R_{16}$ | jeweils unabhängig voneinander für Wasserstoff oder ein Halogenatom oder ein Pseudohalogen oder eine Hydroxygruppe oder eine Amingruppe oder eine Nitrogruppe oder Alkylgruppe mit 1 bis 20 Kohlenstoffatomen oder eine Cycloalkylgruppe mit 1 bis 20 Kohlenstoffatomen oder eine Alkenylgruppe mit 2 bis 20 Kohlenstoffatomen oder eine Alkinylgruppe mit 2 bis 20 Kohlenstoffatomen oder eine Arylgruppe oder eine Alkoxygruppe mit 1 bis 20 Kohlenstoffatomen oder eine Acylgruppe mit 1 bis 20 Kohlenstoffatomen oder eine Aldehydgruppe oder eine Carbonsäuregruppe oder eine Carbonsäureestergruppe mit 1 bis 20 Kohlenstoffatomen oder eine Amidgruppe oder eine Acylchlorid-, Acylbromid-, Acyliodid- oder Acylfluorid-Gruppe, stehen, |
| m, n | jeweils unabhängig voneinander für eine Zahl ≥ 0 bis ≤ 10, beispielsweise ≥ 0 bis ≤ 2, insbesondere 0 oder 1, stehen, und |
| $T_1$, $T_2$ | jeweils unabgängig voneinander für eine organische Gruppe stehen. |

Im Rahmen der vorliegenden Erfindung hat es sich als vorteilhaft herausgestellt, wenn das Molekulargewicht der Verbindung der allgemeinen Formel (I) ≥ 500 g/mol beträgt, da hierdurch eine, insbesondere übermäßige, Verflüchtigung der Verbindung der allgemeinen Formel (I) während der Verarbeitung der erfindungsgemäßen Zusammensetzung vermieden wird.

Beispielsweise können $R_1$, $R_2$, $R_3$, $R_4$, $R_5$, $R_6$, $R_{13}$, $R_{14}$, $R_{15}$, $R_{16}$ jeweils unabhängig voneinander ein Fluor-, Chlor-, Brom- oder Iodatom, vorzugsweise ein Fluoratom, sein.

Im Rahmen einer Ausführungsform der vorliegenden Erfindung stehen $R_1$, $R_2$, $R_3$, $R_4$, $R_5$, $R_6$, $R_{13}$, $R_{14}$, $R_{15}$, $R_{16}$ jeweils unabhängig voneinander für eine halogenierte, beispielsweise fluorierte, insbesondere perfluorierte, Alkyl-, Cycloalkyl-, Alkenyl- Alkinyl-, Aryl-, Alkoxy-, Acyl- oder Carbonsäureester-Gruppe.

Im Rahmen einer bevorzugten Ausführungsform stehen $R_1$ und $R_2$ jeweils unabhängig voneinander für ein Fluoratom oder ein Pseudohalogen oder eine Hydroxygruppe oder eine Amingruppe oder eine Nitrogruppe oder eine fluorierte Alkylgruppe mit 1 bis 20 Kohlenstoffatomen oder eine fluorierte Cycloalkylgruppe mit 1 bis 20 Kohlenstoffatomen oder eine fluorierte Alkenylgruppe mit 2 bis 20 Kohlenstoffatomen oder eine fluorierte Alkinylgruppe mit 2 bis 20 Kohlenstoffatomen oder eine fluorierte Arylgruppe oder eine fluorierte Alkoxygruppe mit 1 bis 20 Kohlenstoffatomen oder eine fluorierte Acylgruppe mit 1 bis 20 Kohlenstoffatomen oder eine Aldehydgruppe oder eine Carbonsäuregruppe oder eine fluorierte Carbonsäureestergruppe mit 1 bis 20 Kohlenstoffatomen oder eine Amidgruppe oder eine Acylchlorid- oder Acylfluorid-Gruppe.

Im Rahmen einer besonders bevorzugten Ausführungsform stehen $R_1$ und $R_2$ jeweils unabhängig voneinander für eine Nitrogruppe oder eine Trifluormethylgruppe oder eine Pentafluorethylgruppe oder eine Heptafluor-n-propylgruppe oder eine Heptafluorisopropylgruppe oder eine Nonafluor-n-butylgruppe oder eine Nonafluortertbutylgruppe, insbesondere eine Trifluormethylgruppe.

$R_6$ und $R_{16}$ können im Rahmen der vorliegenden Erfindung jeweils unabhängig voneinander für Wasserstoff oder eine substituierte oder unsubstituierte, halogenierte oder unhalogenierte Arylgruppe, beispielsweise eine Phenylgruppe, stehen.

Im Rahmen der vorliegenden Erfindung können $T_1$ und $T_2$ jeweils unabgängig voneinander für eine organische Gruppe mit einem Molekulargewicht von ≥ 75 g/mol, beispielsweise von ≥ 150 g/mol, insbesondere von ≥ 300 g/mol, stehen. Beispielsweise können $T_1$ und $T_2$ jeweils unabgängig voneinander für eine organische Gruppe mit mindestens 6 Kohlenstoffatomen, insbesondere für eine substituierte oder unsubstituierte, halogenierte oder unhalogenierte Aryl- oder Aryloxygruppe mit mindestens 6 Kohlenstoffatomen stehen.

Im Rahmen einer bevorzugten Ausführungsform der vorliegenden Erfindung stehen $T_1$ für

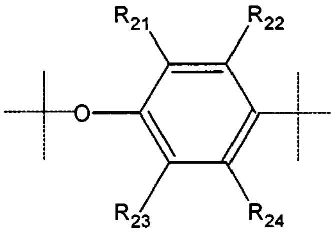 oder 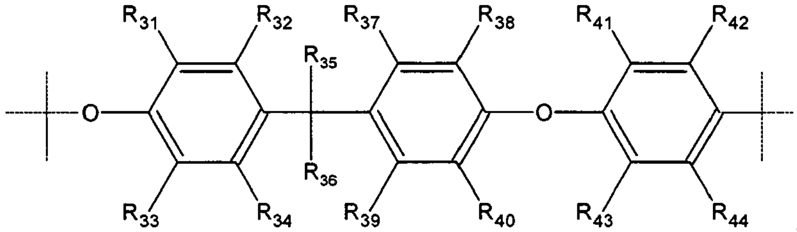 ,

und $T_2$ für

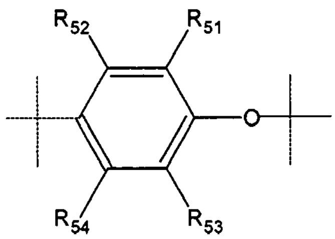 oder 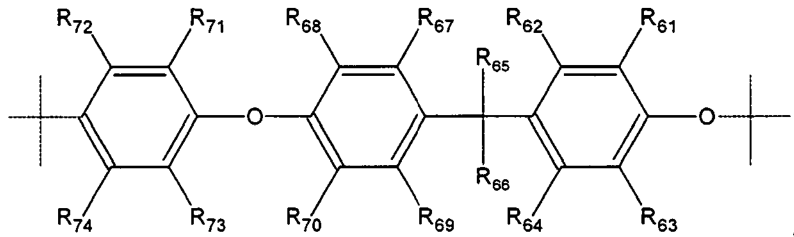 ,

wobei $R_{21}$ bis $R_{74}$ jeweils unabhängig voneinander für Wasserstoff oder ein Halogenatom oder ein Pseudohalogen oder eine Hydroxygruppe oder eine Amingruppe oder eine Nitrogruppe oder Alkylgruppe mit 1 bis 20 Kohlenstoffatomen oder eine Cycloalkylgruppe mit 1 bis 20 Kohlenstoffatomen oder eine Alkenylgruppe mit 2 bis 20 Kohlenstoffatomen oder eine Alkinylgruppe mit 2 bis 20 Kohlenstoffatomen oder eine Arylgruppe oder eine Alkoxygruppe mit 1 bis 20 Kohlenstoffatomen oder eine Acylgruppe mit 1 bis 20 Kohlenstoffatomen oder eine Aldehydgruppe oder eine Carbonsäuregruppe oder eine Carbonsäureestergruppe mit 1 bis 20 Kohlenstoffatomen oder eine Amidgruppe oder eine Acylchlorid-, Acylbromid-, Acyliodid- oder Acylfluorid-Gruppe, stehen.

Im Rahmen der vorliegenden Erfindung symbolisiert das Zeichen

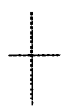

eine Verbindung mit den jeweiligen durch $T_1$ beziehungsweise $T_2$ verbrückten Gruppe der Verbindung der allgemeinen Formel (I), insbesondere

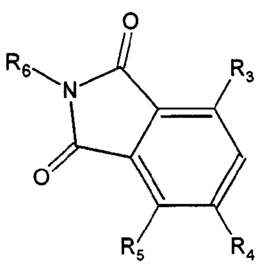 beziehungsweise 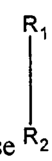 beziehungsweise 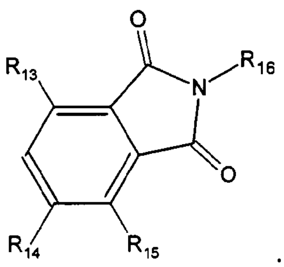 .

Vorzugsweise umfasst die Verbindung der allgemeinen Formel (I) die bevorzugten Gruppen von $T_1$ und/oder

$T_2$ in der dargestellten Ausrichtung. Mit anderen Worten, wenn beispielsweise m 1, n 0 und $T_1$

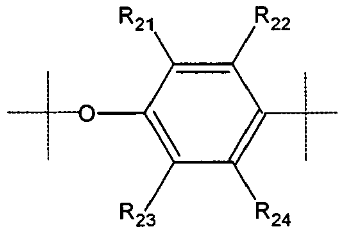

wäre, dann entspräche dies vorzugsweise:

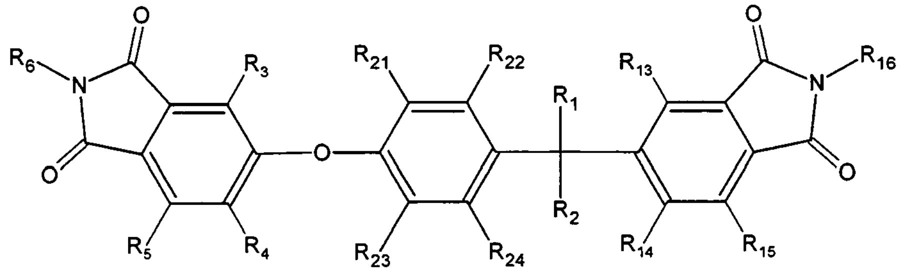

und nicht

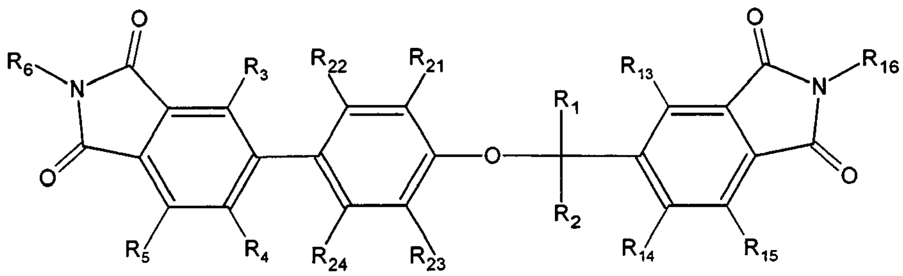

Beispielsweise können $R_{21}$ bis $R_{74}$ jeweils unabhängig voneinander ein Fluor-, Chlor-, Brom- oder Iodatom, insbesondere ein Fluoratom, sein.

Im Rahmen einer Ausführungsform der vorliegenden Erfindung stehen $R_{21}$ bis $R_{74}$ jeweils unabhängig voneinander für eine halogenierte, beispielsweise fluorierte, insbesondere perfluorierte, Alkyl-, Cycloalkyl-, Alkenyl- Alkinyl-, Aryl-, Alkoxy-, Acyl- oder Carbonsäureester-Gruppe.

Im Rahmen der vorliegenden Erfindung hat sich vorteilhafterweise herausgestellt, dass die Elektreteigenschaften eines Polycarbonats auch dann durch eine Verbindung der allgemeinen Formel (I) verbessert werden, wenn nur $R_1$ und $R_2$ und/oder $R_{35}$ und $R_{36}$ und/oder $R_{65}$ und $R_{66}$ halogeniert, beispielsweise fluoriert, insbesondere perfluoriert, sind. $R_3$, $R_4$, $R_5$, $R_6$, $R_{13}$, $R_{14}$, $R_{15}$, $R_{16}$, $R_{21}$ bis $R_{24}$, $R_{31}$ bis $R_{34}$, $R_{37}$ bis $R_{44}$, $R_{51}$ bis $R_{54}$, $R_{61}$ bis $R_{64}$, $R_{67}$ bis $R_{74}$ können daher im Rahmen der vorliegenden Erfindung unhalogeniert, insbesondere unfluoriert, sein.

Beispielsweise können $R_1$ und $R_2$ jeweils unabhängig voneinander für eine Nitrogruppe oder eine Trifluormethylgruppe, insbesondere eine Trifluormethylgruppe, und/oder $R_3$, $R_4$, $R_5$, $R_{13}$, $R_{14}$, $R_{15}$ für Wasserstoff und/oder $R_6$ und $R_{16}$ für eine Phenylgruppe und/oder $T_1$ für

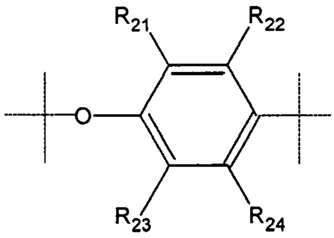

oder

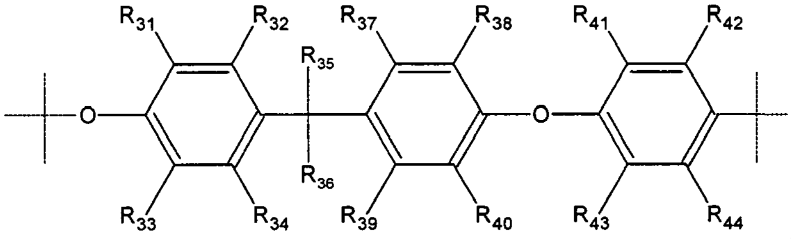

,

und/oder $T_2$ für

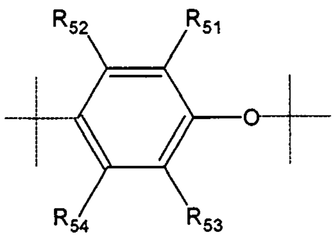

oder

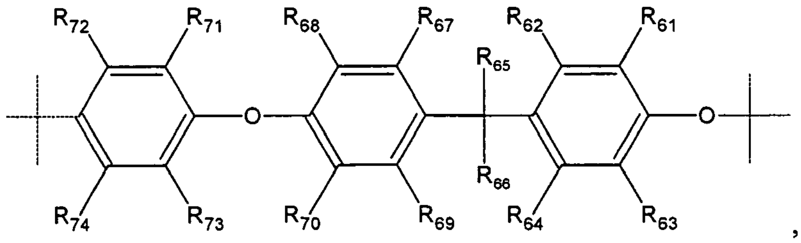

,

stehen, wobei $R_{21}$ bis $R_{24}$ oder $R_{31}$ bis $R_{34}$ und $R_{37}$ bis $R_{44}$ und/oder $R_{51}$ bis $R_{54}$ oder $R_{61}$ bis $R_{64}$ und $R_{67}$ bis $R_{74}$ für Wasserstoff und/oder $R_{35}$ und $R_{36}$ und/oder $R_{65}$ und $R_{66}$ jeweils unabhängig voneinander für eine Nitrogruppe oder eine Trifluormethylgruppe, insbesondere eine Trifluormethylgruppe, stehen.

Beispiele für Verbindungen der allgemeinen Formel (I) sind:

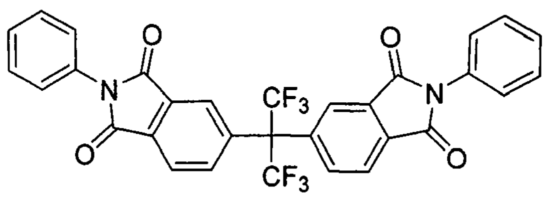

,

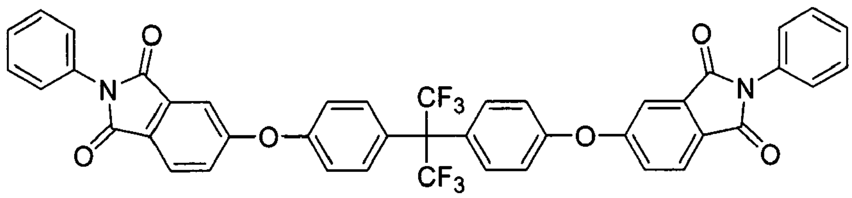

und

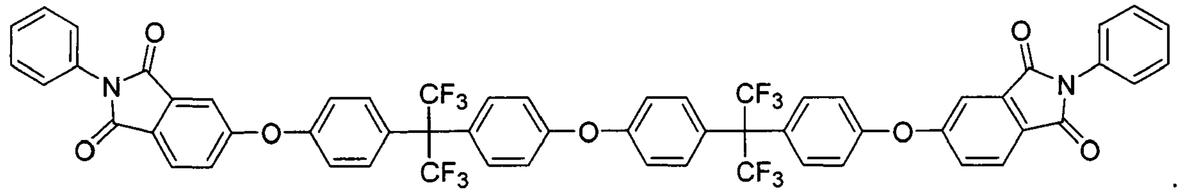

.

Im Rahmen der vorliegenden Erfindung beträgt der Anteil der Zusammensetzung an Verbindungen der allgemeinen Formel (I) vorzugsweise ≥ 0,01 Gew.-% bis ≤ 1 Gew.-% (≥ 100 Gew.-ppm bis ≤ 10000 Gew.-ppm), besonders

bevorzugt ≥ 0,02 Gew.-% bis ≤ 0,15 Gew.-% (≥ 200 Gew.-ppm bis ≤ 1500 Gew.-ppm), ganz besonders bevorzugt ≥ 0,03 Gew.-% bis ≤ 0,11 Gew.-% (≥ 300 Gew.-ppm bis ≤ 1100 Gew.-ppm) bezogen auf das Gesamtgewicht der Zusammensetzung.

Ein weiterer Gegenstand der vorliegenden Erfindung betrifft eine Verbindung der allgemeinen Formel (I)

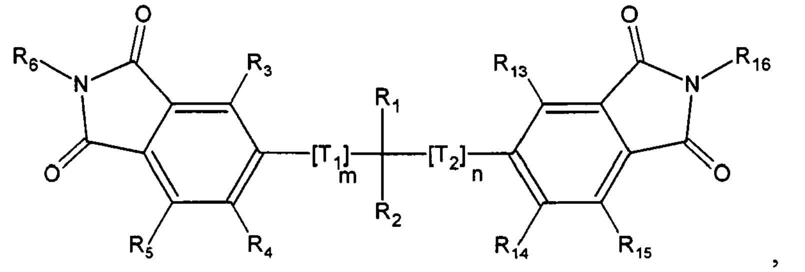

,

wobei

m, n jeweils unabhängig voneinander für eine Zahl ≥ 1 bis ≤ 10, beispielsweise 1 oder 2, insbesondere 1, stehen, und

$T_1$ für

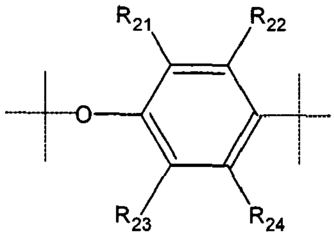

oder

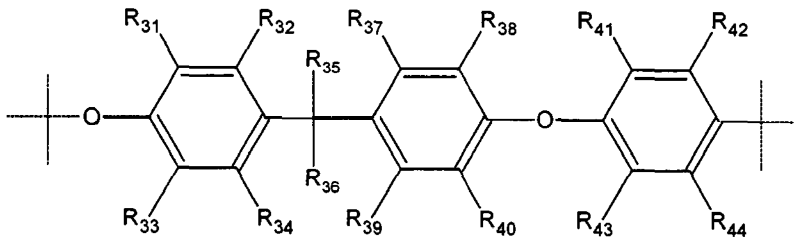

steht, und $T_2$ für

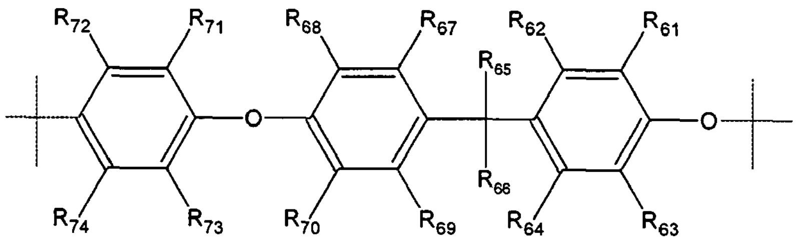

steht,

wobei $R_1$, $R_2$, $R_3$, $R_4$, $R_5$, $R_6$, $R_{13}$, $R_{14}$, $R_{15}$, $R_{16}$, $R_{21}$ bis $R_{74}$ jeweils unabhängig voneinander für Wasserstoff oder ein Halogenatom oder ein Pseudohalogen oder eine Hydroxygruppe oder eine Amingruppe oder eine Nitrogruppe oder Alkylgruppe mit 1 bis 20 Kohlenstoffatomen oder eine Cycloalkylgruppe mit 1 bis 20 Kohlenstoffatomen oder eine Alkenylgruppe mit 2 bis 20 Kohlenstoffatomen oder eine Alkinylgruppe mit 2 bis 20 Kohlenstoffatomen oder eine Arylgruppe oder eine Alkoxygruppe mit 1 bis 20 Kohlenstoffatomen oder eine Acylgruppe mit 1 bis 20 Kohlenstoffatomen oder eine Aldehydgruppe oder eine Carbonsäuregruppe oder eine Carbonsäureestergruppe mit 1 bis 20 Kohlenstoffatomen oder eine Amidgruppe oder eine Acylchlorid-, Acylbromid-, Acyliodid- oder Acylfluorid-Gruppe, stehen.

Hinsichtlich spezieller Beispiele und Ausführungsformen der Gruppen: $R_1$, $R_2$, $R_3$, $R_4$, $R_5$, $R_6$, $R_{13}$, $R_{14}$, $R_{15}$, $R_{16}$, $T_1$, $T_2$, $R_{21}$ bis $R_{74}$, wird hiermit explizit auf die Ausführungen im Zusammenhang mit der erfindungsgemäßen Zusammensetzung verwiesen.

Beispielsweise betrifft die Erfindung eine Verbindung der Formel:

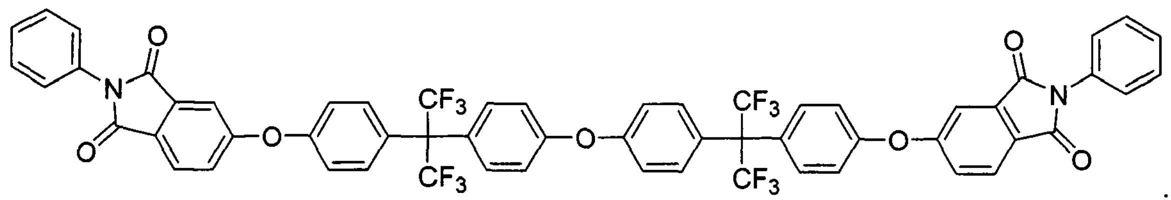

.

Ein weiterer Gegenstand der vorliegenden Erfindung betrifft ein Polymerelement, umfassend eine erfindungsgemäße Zusammensetzung, beispielsweise ein Polymerelement erhältlich aus einer erfindungsgemäßen Zusammensetzung. Im Rahmen der vorliegenden Erfindung kann es sich bei dem Polymerelement um eine Polymerschicht, insbesondere einen Polymerfilm, eine Polymermembran, eine Polymerfolie oder eine Polymerbeschichtung handeln. Beispielsweise kann die Polymerschicht eine Schichtdicke von ≥ 0,1 µm bis ≤ 1500 µm, beispielsweise von > 10 µm bis ≤ 500 µm, insbesondere von ≥ 10 µm bis ≤ 150 µm, aufweisen. Im Rahmen einer bevorzugten Ausführungsform der vorliegenden Erfindung ist das Polymerelement ein Polymerfilm.

Ein weiterer Gegenstand der vorliegenden Erfindung betrifft ein Verfahren zur Herstellung eines erfindungsgemäßen Polymerelements, in dem das Polymerelement aus einer erfindungsgemäßen Zusammensetzung hergestellt wird.

Das Polymerelement kann beispielsweise durch Pressen, insbesondere Schmelzpressen, Rakeln, insbesondere Lösungsrakeln, Streichen, Schleudern, insbesondere Lackschleudern, Sprühen, Extrusion, insbesondere Folienextrusion, Blasformen, insbesondere Folienblasen, und/oder Kalandern hergestellt werden. Vorzugsweise wird das Polymerelement dabei durch Schmelzpressen, Lösungsrakeln, Folienextrusion oder Folienblasen und/oder Kalandern hergestellt. Das Polycarbonat und die Verbindung/en der allgemeinen Formel (I) können dabei vorgemischt, und das resultierende Gemisch, insbesondere Pulvergemisch, anschließend schmelzextrudiert werden. Das Polycarbonat und die Verbindung/en der allgemeinen Formel (I) können jedoch auch vorgemischt und granuliert werden, und das resultierende Granulat anschließend schmelzextrudiert werden. Im Rahmen einer weiteren Ausführungsform können die Verbindung/en der allgemeinen Formel (I) erst im Extruder mit dem Polycarbonat vermischt und dann schmelzextrudiert werden. Die Extrusionsbedingungen können dabei den Extrusionsbedingungen entsprechen, welche zum Extrudieren des Polycarbonats ohne Additiv geeignet sind.

Im Rahmen einer bevorzugten Ausführungsform der vorliegenden Erfindung wird das Polymerelement getempert. Das Polymerelement kann dabei unterhalb, bei oder oberhalb der Glasübergangstemperatur des Polycarbonats getempert werden. Beispielsweise kann das Polymerelement für ≥ 5 min bis ≤ 24 h, bevorzugt für ≥ 10 min bis ≤ 12 h bei ≥ 90 °C bis ≤ 200 °C, bevorzugt bei ≥ 120 °C bis ≤ 180 °C, getempert werden.

Ein weiterer Gegenstand der vorliegenden Erfindung betrifft ein durch das zuvor beschriebene, erfindungsgemäße Verfahren hergestelltes Polymerelement.

Die vorliegende Erfindung betrifft weiterhin ein Elektretelement, welches ein aufgeladenes erfindungsgemäßes Polymerelement umfasst. Im Rahmen der vorliegenden Erfindung kann es sich bei dem Elektretelement um eine Elektretschicht, insbesondere einen Elektretfilm, eine Elektretmembran, eine Elektretfolie oder eine Elektretbeschichtung handeln. Beispielsweise kann die Elektretschicht eine Schichtdicke von ≥ 0,1 1 µm bis ≤ 1500 µm, beispielsweise von ≥ 10 µm bis ≤ 500 µm, insbesondere von ≥ 10 µm bis ≤ 150 µm, aufweisen. Im Rahmen einer bevorzugten Ausführungsform der vorliegenden Erfindung ist das Elektretelement ein Elektretfilm.

Ein weiterer Gegenstand der vorliegenden Erfindung betrifft ein Verfahren zur Herstellung eines erfindungsgemäßen Elektretelements, in dem ein erfindungsgemäßes Polymerelement aus einer erfindungsgemäßen Zusammensetzung hergestellt und aufgeladen wird.

Das Elektretelement kann dabei beispielsweise durch Pressen, insbesondere Schmelzpressen, Rakeln, insbesondere Lösungsrakeln, Streichen, Schleudern, insbesondere Lackschleudern, Sprühen, Extrusion, insbesondere Folienextrusion, Blasformen, insbesondere Folienblasen, und/oder Kalandern hergestellt werden. Vorzugsweise wird das Polymerelement dabei durch Schmelzpressen, Lösungsrakeln, Folienextrusion oder Folienblasen und/oder Kalandern hergestellt. Das Polycarbonat und die Verbindung/en der allgemeinen Formel (I) können dabei vorgemischt, und das resultierende Gemisch, insbesondere Pulvergemisch, anschließend schmelzextrudiert werden. Das Polycarbonat und die Verbindung/en der allgemeinen Formel (I) können jedoch auch vorgemischt und granuliert werden, und das resultierende Granulat anschließend schmelzextrudiert werden. Im Rahmen einer weiteren Ausführungsform können die Verbindung/en der allgemeinen Formel (I) erst im Extruder mit dem Polycarbonat vermischt und dann schmelzextrudiert werden. Die Extrusionsbedingungen können dabei den Extrusionsbedingungen entsprechen, welche zum Extrudieren des Polycarbonats ohne Additiv geeignet sind.

Das Aufladen des Polymerelements kann im Rahmen der vorliegenden Erfindung durch Triboaufladung, Elektronenstrahlbeschuss, Abkühlen der Polymerschmelze im elektrischen Feld, Anlegen einer elektrischen Spannung oder Coronoentladung erfolgen. Im Rahmen einer bevorzugten Ausführungsform dieses Verfahrens erfolgt das Aufladen im Rahmen der vorliegenden Erfindung durch Coronaentladung.

Im Rahmen einer weiteren bevorzugten Ausführungsform dieses Verfahrens wird das Polymerelement vor dem Aufladen getempert. Vorzugsweise wird das Polymerelement vor dem Aufladen getempert. Beispielsweise kann das Polymerelement für ≥ 5 min bis ≤ 24 h, bevorzugt für ≥ 10 min bis ≤ 12 h bei ≥ 90 °C bis ≤ 200 °C, bevorzugt bei ≥ 120 °C bis ≤ 180 °C, vor dem Aufladen getempert werden.

Ein weiterer Gegenstand der vorliegenden Erfindung betrifft ein durch das zuvor beschriebene, erfindungsgemäße Verfahren hergestelltes Elektretelement.

Die vorliegende Erfindung betrifft weiterhin einen elektromechanischen, beispielsweise piezoelektrischen, Wandler, der ein erfindungsgemäßes Polymerelement oder ein erfindungsgemäßes Elektretelement oder ein durch das erfindungsgemäße Verfahren hergestelltes Polymerelement oder ein durch das erfindungsgemäße Verfahren hergestelltes Elektretelement, umfasst.

Ein weiterer Gegenstand der vorliegenden Erfindung ist die Verwendung einer Verbindung der allgemeinen Formel (I):

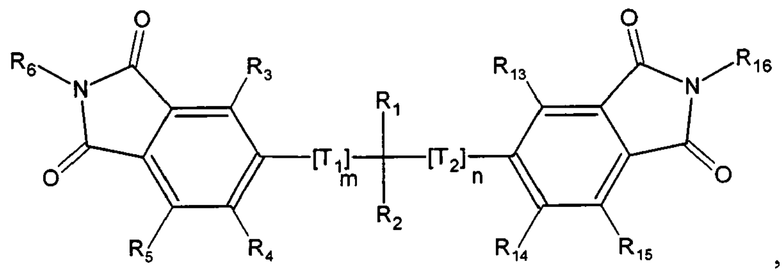

,

wobei

| | |
|---|---|
| $R_1$, $R_2$, $R_3$, $R_4$, $R_5$, $R_6$, $R_{13}$, $R_{14}$, $R_{15}$, $R_{16}$ | jeweils unabhängig voneinander für Wasserstoff oder ein Halogenatom oder ein Pseudohalogen oder eine Hydroxygruppe oder eine Amingruppe oder eine Nitrogruppe oder Alkylgruppe mit 1 bis 20 Kohlenstoffatomen oder eine Cycloalkylgruppe mit 1 bis 20 Kohlenstoffatomen oder eine Alkenylgruppe mit 2 bis 20 Kohlenstoffatomen oder eine Alkinylgruppe mit 2 bis 20 Kohlenstoffatomen oder eine Arylgruppe oder eine Alkoxygruppe mit 1 bis 20 Kohlenstoffatomen oder eine Acylgruppe mit 1 bis 20 Kohlenstoffatomen oder eine Aldehydgruppe oder eine Carbonsäuregruppe oder eine Carbonsäureestergruppe mit 1 bis 20 Kohlenstoffatomen oder eine Amidgruppe oder eine Acylchlorid-, Acylbromid-, Acyliodid- oder Acylfluorid-Gruppe, stehen, und |
| m, n | jeweils unabhängig voneinander für eine Zahl ≥ 0 bis ≤ 10, beispielsweise ≥ 0 bis ≤ 2, insbesondere 0 oder 1, stehen, und |
| $T_1$, $T_2$ | jeweils unabgängig voneinander für eine organische Gruppe stehen, |

als Additiv zur Verbesserung der Elektreteigenschaften eines Polycarbonats.

Hinsichtlich spezieller Beispiele und Ausführungsformen der Gruppen: $R_1$, $R_2$, $R_3$, $R_4$, $R_5$, $R_6$, $R_{13}$, $R_{14}$, $R_{15}$, $R_{16}$, $T_1$, $T_2$, $R_{21}$ bis $R_{74}$ wird hiermit explizit auf die Ausführungen im Zusammenhang mit der erfindungsgemäßen Zusammensetzung verwiesen.

Die vorliegende Erfindung betrifft ferner die Verwendung eines erfindungsgemäßen Polymerelements und/oder eines erfindungsgemäßen Elektretelements und/oder eines durch das erfindungsgemäße Verfahren hergestellten Polymerelements und/oder eines durch das erfindungsgemäße Verfahren hergestellten Elektretelements in einer elektromechanischen, beispielsweise piezoelektrischen, Anordnung, beispielsweise in einem Sensor, Aktuator und/oder Generator, insbesondere in einem Mikrofon oder Lautsprecher, beispielsweise als akustische Membran, in einem

Schwingungswandler, in einem pyroelektrischen Detektor oder in einem Kondensator.

Weiterhin betrifft die vorliegende Erfindung die Verwendung eines erfindungsgemäßen Polymerelements und/oder eines erfindungsgemäßen Elektretelements und/oder eines durch das erfindungsgemäße Verfahren hergestellten Polymerelements und/oder eines durch das erfindungsgemäße Verfahren hergestellten Elektretelements als selbsthaftender Film, beispielsweise im Designbereich, in der Plakatwerbung oder für Schilder. Die erfindungsgemäßen Polymerelemente und/oder Elektretelemente haben dabei den Vorteil, dass diese sowohl rückstandslos ablösbar als auch wiederverwendbar sein können.

Darüber hinaus können die erfindungsgemäßen Polymerelemente und/oder Elektretelemente und/oder durch das erfindungsgemäße Verfahren hergestellte Polymerelemente und/oder durch das erfindungsgemäße Verfahren hergestellte Elektretelemente zu Fasern, Geweben, Netzen, gelochten Filmen und Kompositen weiterverarbeitet werden, die ihrerseits als Filtermaterialien, Wundverbände oder in elektromechanischen, beispielsweise piezoelektrischen, Anordnungen Verwendung finden.

**Beispiele**

Beispiel 1: Herstellung eines ersten erfindungsgemäßen Additivs

In einen Schlenkkolben mit Rührer, Gasein- und Gasauslass wurden 65 g Benzoesäure eingewogen. Dazu wurden 4,4'-(Hexafluoroisopropyliden)diphthalsäureanhydrid (5,33 g, 12 mmol) und Anilin (2,52 ml, 27,6 mmol) gegeben. Das Gemisch wurde im heißen Ölbad unter leichtem Argonfluss binnen 5 min auf 150 °C erwärmt, so dass die gesamte Benzoesäure aufgeschmolzen war. Unter leichtem Argonfluss wurde für eine Stunde bei 150 °C gerührt und sodann die Lösung unter Rühren in kalte Natronlauge (0,5 M, 600 ml) gegeben. Anschließend wurde die wässrige Phase mit Chloroform ausgeschüttelt. Die vereinigten organischen Phasen wurden am Rotationsverdampfer eingedampft und der verbliebene Rückstand aus Ethanol umkristallisiert. Anschließend wurde im Vakuum bei 80 °C für 12 h getrocknet. Dabei erhielt man 6,13 g eines weißen Feststoffs der Struktur

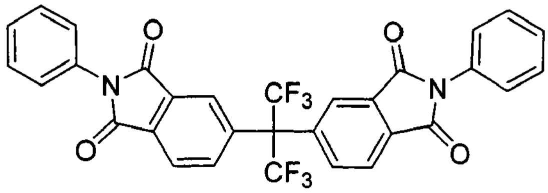

,

welche über Kernresonanzspektroskopie bestätigt wurde.

Beispiel 2: Herstellung eines zweiten erfindungsgemäßen Additivs

In einen Schlenkkolben mit Rührer, Gasein- und Gasauslass wurden 11 g Benzoesäure eingewogen. Dazu gab man 4,4'-(4,4'-Hexafluoroisopropyliden-diphenoxy)-bis-(phthal-säure-anhydrid) (943 mg, 1,5 mmol) und Anilin (0,32 ml, 3,45 mmol). Das Gemisch wurde im heißen Ölbad unter leichtem Argonfluss binnen 5 min auf 150 °C erwärmt, so dass die gesamte Benzoesäure aufgeschmolzen war. Unter leichtem Argonfluss wurde für eine Stunde bei 150 °C gerührt und sodann die Lösung unter Rühren in kalte Natronlauge (0,5 M, 120 ml) gegeben. Anschließend wurde die wässrige Phase gegen Dichlormethan ausgeschüttelt. Die vereinigten organischen Phasen wurden am Rotationsverdampfer eingedampft, der Rückstand in wenig Tetrahydrofuran gelöst und die Lösung in Wasser gefällt. Nach dem Einengen der Lösung wurde diese im Kühlschrank auf 5 °C gekühlt. Der Rückstand wurde abfiltriert, mit etwas Wasser nachgewaschen und im Vakuum bei 80 °C für 72 h getrocknet. Es wurden 876 mg eines weißen Feststoffs der Struktur

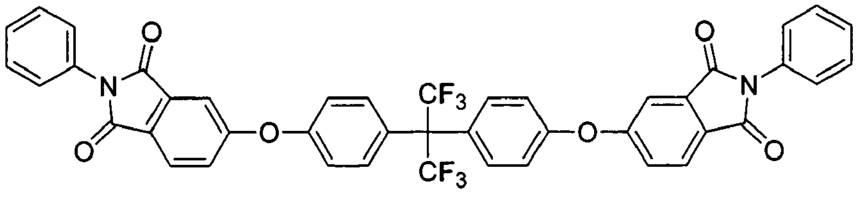

erhalten, die über Kernresonanzspektroskopie bestätigt wurde und einen Schmelzpunkt von 217°C besitzt.

Beispiel 3a bis 3f: Herstellung von sechs erfmdungsgemäßen Elektretelementen

Das Produkt aus Beispiel 1 wurde mit Makrolon® 3108-Pulver (15,97 g) gemischt, wobei sich ein Additivgehalt von 0,2 Gew.-% ergab. Dieses Gemisch wurde in einem Doppelschneckenmischer (Technical University of Eindhoven) bei 280 °C für 3 min gemischt. Durch Extrusion eines Teils der Polymerschmelze und anschließendem Hinzufügen von reinem Makrolon® 3108-Pulver in den Mischer erstellte man eine Reihe von Extrudaten mit den Additiv-Konzentrationen (in Gew.-ppm):

| | |
|---|---|
| Beispiel 3a: | 2000 ppm |
| Beispiel 3b: | 1100 ppm |
| Beispiel 3c: | 610 ppm |
| Beispiel 3d: | 350 ppm |
| Beispiel 3e: | 210 ppm |
| Beispiel 3f: | 130 ppm |

Die Granulatstränge wurden anschließend granuliert und aus dem Granulat wurden Filme von 100 μm Dicke in einer P.O. Weber PW10 Laborpresse bei 230 °C schmelzgepresst.
Die Polymerfilme wurden anschließend in einer Corona-Apparatur 10s (Coronaexposition) aufgeladen.
Dazu wurden im Rahmen dieser Beispiele als auch der folgenden Beispiele etwa 4x4 $cm^2$ große Filme (100 μm Dicke) mittels eines leitfähigen, doppelseitigen Klebebands auf eine Aluminiumplatte geklebt. Diese Proben wurden bei Raumtemperatur in einer Nadel-Platte-Corona-Apparatur auf +400 V aufgeladen. Die Coronaspannung beziehungsweise Nadelspannung betrug hierbei +12,5 kV und die Gitterspannung, die zu einer homogenen Ladungsverteilung auf der Filmoberfläche führen soll, betrug +400 V.

Beispiel 4: Herstellung eines ersten Vergleichselektretelements

Für das Vergleichselektretelement V1 wurden Filme von 100 μm Dicke aus reinem, das heißt ohne jegliche Zusatzstoffe, Makrolon® 3108-Pulver durch Mischen im Doppelschneckenmischer und anschließendem Schmelzpressen der granulierten Extrudatstränge hergestellt.
Die Vergleichselektretfilme wurden in einer Corona-Apparatur 10s aufgeladen.

Beispiel 5a bis 5f: Herstellung von weiteren sechs erfindungsgemäßen Elektretelementen

Das Produkt aus Beispiel 2 wurde mit Makrolon® 3108-Pulver (15,97 g) gemischt, wobei sich ein Additivgehalt von 0,2 Gew.-% ergab. Dieses Gemisch wurde in einem Doppelschneckenmischer (Technical University of Eindhoven) bei 280 °C für 3 min gemischt. Durch Extrusion eines Teils der Polymerschmelze und anschließendem Hinzufügen von reinem Makrolon® 3108-Pulver in den Mischer erstellte man eine Reihe von Extrudaten mit den Additiv-Konzentrationen (in Gew.-ppm):

| | |
|---|---|
| Beispiel 5a: | 2000 ppm |
| Beispiel 5b: | 1100 ppm |
| Beispiel 5c: | 590 ppm |
| Beispiel 5d: | 330 ppm |
| Beispiel 5e: | 190 ppm |
| Beispiel 5f: | 110 ppm |

Die Granulatstränge wurden anschließend granuliert und aus dem Granulat wurden Filme von 100 μm Dicke in einer P.O. Weber PW10 Laborpresse bei 230 °C schmelzgepresst.
Die Polymerfilme wurden anschließend in einer Corona-Apparatur für 10 s aufgeladen.

Beispiel 6a bis 6f: Herstellung von weiteren sechs erfmdungsgemäßen Elektretelementen

Das Produkt aus Beispiel 2 wurde mit Makrolon® 3108-Pulver (15,97 g) gemischt, wobei sich ein Additivgehalt von 0,2 Gew.-% ergab. Dieses Gemisch wurde in einem Doppelschneckenmischer (Technical University of Eindhoven)

bei 280 °C für 3 min gemischt. Durch Extrusion eines Teils der Polymerschmelze und anschließendem Hinzufügen von reinem Makrolon® 3108-Pulver in den Mischer erstellte man eine Reihe von Extrudaten mit den Additiv-Konzentrationen (in Gew.-ppm):

| | |
|---|---|
| Beispiel 6a: | 2000 ppm |
| Beispiel 6b: | 1100 ppm |
| Beispiel 6c: | 590 ppm |
| Beispiel 6d: | 330 ppm |

Die Granulatstränge wurden anschließend granuliert und aus dem Granulat wurden Filme von 100 $\mu$m Dicke in einer P.O. Weber PW10 Laborpresse bei 230 °C schmelzgepresst.
Die Polymerfilme wurden anschließend für 12 h bei 150°C getempert.
Danach wurden die Polymerfilme in einer Corona-Apparatur für 30 s aufgeladen.

Beispiel 7: Herstellung eines zweiten Vergleichselektretelements

Für das Vergleichselektretelement V2 wurden Filme von 100 $\mu$m Dicke aus reinem, das heißt ohne jegliche Zusatzstoffe, Makrolon® 3108-Pulver durch Mischen im Doppelschneckenmischer und anschließendem Schmelzpressen der granulierten Extrudatstränge hergestellt.
Die Polymerfilme wurden anschließend für 12 h bei 150°C getempert.
Danach wurden die Vergleichselektretfilme in einer Corona-Apparatur für 30 s aufgeladen.

Beispiel 8a bis 8c: Herstellung von weiteren drei erfindungsgemäßen Elektretelementen

Das Produkt aus Beispiel 2 wurde mit Makrolon® 3108-Pulver (15,97 g) gemischt, wobei sich ein Additivgehalt von 0,2 Gew.-% ergab. Dieses Gemisch wurde in einem Doppelschneckenmischer (Technical University of Eindhoven) bei 280 °C für 3 min gemischt. Durch Extrusion eines Teils der Polymerschmelze und anschließendem Hinzufügen von reinem Makrolon® 3108-Pulver in den Mischer erstellte man eine Reihe von Extrudaten mit den Additiv-Konzentrationen (in Gew.-ppm):

| | |
|---|---|
| Beispiel 8a: | 1100 ppm |
| Beispiel 8b: | 590 ppm |
| Beispiel 8c: | 330 ppm |

Die Granulatstränge wurden anschließend granuliert und aus dem Granulat wurden Filme von 100 $\mu$m Dicke in einer P.O. Weber PW10 Laborpresse bei 230 °C schmelzgepresst.
Die Polymerfilme wurden anschließend für 12 h bei 150°C getempert.
Danach wurden die Polymerfilme in einer Corona-Apparatur für 30 s aufgeladen.

Bewertung der erfindungsgemäßen Elektretelemente (Beispiel 3a-3f, 5a-5f, 6a-6f, 8a-8c)

Zur Bewertung der erfindungsgemäßen Elektretfilme wurde der isotherme Oberflächenpotentialabfall (ITPD) der erfindungsgemäßen Elektretelemente und der Vergleichselektretelemente V1 und V2 bestimmt. Dazu wurde nach dem Aufladen der Filme das Oberflächenpotential der Filme mit einem elektrostatischen Voltmeter (Monroe Electrostatic Voltmeter 244A) 2 mm über ihrer Oberfläche bestimmt. Hierbei wurden pro Film neun Messpunkte bestimmt und dann der Mittelwert gebildet. Die nach dem Aufladen vorhandene Oberflächenspannung betrug somit für alle Filme +400 V 10 V. Anschließend wurden die Filme bei einer konstanten Temperatur von 90 °C beziehungsweise 120°C im Ofen getempert und nach jeweils 30, 90, 180, 360 und 1440 min für 10 min aus dem Ofen genommen, auf Raumtemperatur abgekühlt und wiederum ihr Oberflächenpotential bestimmt. Aufgrund der besseren Vergleichbarkeit wurden die so erhaltenen ITPD-Kurven nach der folgenden Formel normiert:

$$\text{Normiertes Oberflächenpotential } [V/V_0] = \frac{\text{Oberflächenpotential zum Zeitpunkt } t\ [V]}{\text{Ursprünglich aufgebrachtes Oberfächenpotential } [V_0]}$$

Die Ergebnisse dieser Messungen werden durch die Figuren 1 bis 4 wiedergegeben. Es zeigen:

Figur 1 den isothermen Oberflächenpotentialabfall (ITPD) der erfindungsgemäßen Beispiele 3a bis 3f sowie des Vergleichsbeispiels V1 bei 90 °C;

Figur 2 den isothermen Oberflächenpotentialabfall (ITPD) der erfindungsgemäßen Beispiele 5a bis 5f sowie des Vergleichsbeispiels V1 bei 90 °C;

Figur 3 den isothermen Oberflächenpotentialabfall (ITPD) der erfindungsgemäßen getemperten Beispiele 6a bis 6d sowie des getemperten Vergleichsbeispiels V2 bei 90 °C; und

Figur 4 den isothermen Oberflächenpotentialabfall (ITPD) der erfindungsgemäßen getemperten Beispiele 8a bis 8c sowie des getemperten Vergleichsbeispiels V2 bei 120 °C.

Die Figuren veranschaulichen, dass die in den Beispielen 1 und 2 hergestellten Verbindungen die Ladungsspeichereigenschaften von Polycarbonat positiv beeinflussen.

In Figur 1 sind die Beispiele 3a bis 3f zusammen mit dem Vergleichsbeispiel V1 dargestellt. Die Beispiele 3a bis 3f beinhalten dabei die Verbindung

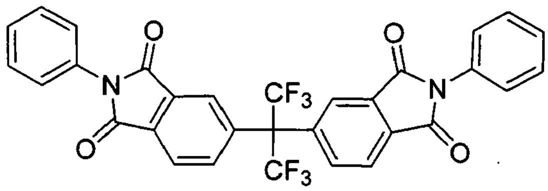

in verschiedenen Konzentrationen in Polycarbonat. Das unadditivierte Vergleichsbeispiel V1 zeigt einen relativ schnellen Ladungsabfall und besitzt nach 24 h des isothermen Temperns bei 90°C nur noch 72 % seiner ursprünglich aufgebrachten Ladung. Die Beispiele 3a bis 3f hingegen zeigen einen im Vergleich zu V1 verminderten Ladungsabfall. Dabei sind nach 24 h des isothermen Temperns bei 90 °C sowohl für das hochadditivierte Beispiel 3a als auch für das geringstadditivierte Bespiel 3f noch 78 % der ursprünglich aufgebrachten Ladung vorhanden, während die Beispiele 3b bis 3e noch 82-83 % besitzen. Mit Additivkonzentrationen zwischen 300 ppm und 1100 ppm sind besonders gute Ladungsretentionen zu beobachten. Mit 83 % Ladungsretention nach 24 h bei 90 °C, zeigt das beste Elektretverhalten Beispiel 3d, welches mit 350 ppm additiviert ist.

Ein ähnliches Elektretverhalten zeigen die in Figur 2 dargestellten Beispiele 5a bis 5f, die in unterschiedlichen Konzentrationen mit der Verbindung

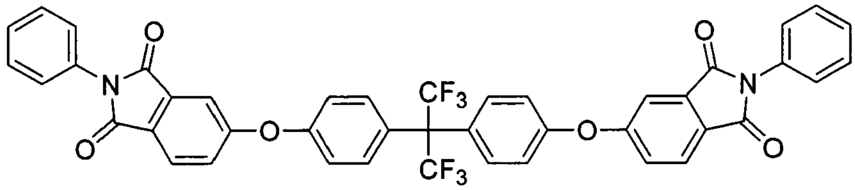

additiviert sind. Die Beispiele zeigen 8 % bis 12 % mehr Ladungsretention nach dem isothermen Tempern bei 90 °C als das Vergleichsbeispiel V1. Mit Additivkonzentrationen zwischen 200 ppm und 600 ppm sind besonders gute Ladungsretentionen zu beobachten. Mit 84 % Ladungsretention nach 24 h bei 90 °C zeigt das beste Elektretverhalten Beispiel 5d, welches mit 330 ppm additiviert ist.

Figur 3 zeigt den Einfluß des Temperns der Filme vor dem Corona-Aufladen an den Beispielen 6a bis 6d sowie dem Vergleichsbeispiel V2. Bereits am unadditivierten Vergleichsbeispiel V2 ist eine deutliche Verbesserung des Elektretverhaltens durch das dem Corona-Aufladeprozeß vorhergehende Tempern zu erkennen. Das Vergleichsbeispiel V2 zeigt einen deutlich gehinderten Ladungsabfall und besitzt nach 24 h des isothermen Temperns bei 90 °C noch 88 % seiner ursprünglich aufgebrachten Ladung, wohingegen das ungetemperte Vergleichsbeispiel V1 nur noch 72 % besitzt.

Durch die Additivierung mit der Verbindung aus Beispiel 2 läßt sich der Ladungserhalt bis um weitere 6 % auf 95 % steigern. Dabei ist im Bereich zwischen 330 ppm und 2000 ppm keine ausgeprägte Konzentrationsabhängigkeit zu erkennen.

In Figur 4 wurde das gleiche Experiment wie in Figur 3 durchgeführt, nur daß das isotherme Tempern für 24 h bei 120 °C stattfand. Für die untersuchten Beispiele 8a bis 8c, welche die gleichen Konzentrationen der Verbindung aus Beispiel 2 wie die Beispiele 6b bis 6d beinhalten, und dem Vergleichsbeispiel V2, ist bei dieser Temperatur ein deutlich beschleunigter Ladungsabfall zu beobachten. Dennoch sind die Beispiel 8a bis 8c nach 24 h des Temperns bis zu 24 % besser als das unadditivierte Vergleichsbeispiel V2. Wiederum läßt sich in dem untersuchten Bereich keine ausgeprägte Abhängigkeit von der Additivkonzentration beobachten. Mit einer Ladungsretention von 50 % nach 24 h bei 120 °C zeigt das beste Elektretverhalten Beispiel 8b, welches mit 590 ppm additiviert ist.

Unabhängig davon, ob die Polymerfilme positiv oder negativ aufgeladen wurden, wurden qualitativ die gleichen Ergebnisse erhalten.

## Patentansprüche

1. Zusammensetzung, umfassend

   - mindestens ein Polycarbonat und
   - mindestens eine Verbindung der allgemeinen Formel (I)

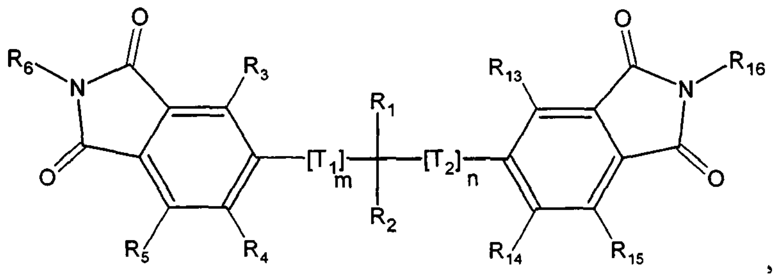

,

wobei

$R_1, R_2, R_3, R_4, R_5, R_6, R_{13}, R_{14}, R_{15}, R_{16}$ jeweils unabhängig voneinander für Wasserstoff oder ein Halogenatom oder ein Pseudohalogen oder eine Hydroxygruppe oder eine Amingruppe oder eine Nitrogruppe oder Alkylgruppe mit 1 bis 20 Kohlenstoffatomen oder eine Cycloalkylgruppe mit 1 bis 20 Kohlenstoffatomen oder eine Alkenylgruppe mit 2 bis 20 Kohlenstoffatomen oder eine Alkinylgruppe mit 2 bis 20 Kohlenstoffatomen oder eine Arylgruppe oder eine Alkoxygruppe mit 1 bis 20 Kohlenstoffatomen oder eine Acylgruppe mit 1 bis 20 Kohlenstoffatomen oder eine Aldehydgruppe oder eine Carbonsäuregruppe oder eine Carbonsäureestergruppe mit 1 bis 20 Kohlenstoffatomen oder eine Amidgruppe oder eine Acylchlorid-, Acylbromid-, Acyliodid- oder Acylfluorid-Gruppe, stehen, und
m, n jeweils unabhängig voneinander für eine Zahl $\geq 0$ bis $\leq 10$ stehen, und
$T_1, T_2$ jeweils unabgängig voneinander für eine organische Gruppe stehen.

2. Zusammensetzung nach Anspruch 1, **dadurch gekennzeichnet, dass**

   $R_1, R_2$ jeweils unabhängig voneinander für eine Nitrogruppe oder eine Trifluormethylgruppe oder eine Pentafluorethylgruppe oder eine Heptafluor-n-propylgruppe oder eine Heptafluorisopropylgruppe oder eine Nonafluor-n-butylgruppe oder eine Nonafluortertbutylgruppe stehen.

3. Zusammensetzung nach Anspruch 1, **dadurch gekennzeichnet, dass**
   $T_1$ für

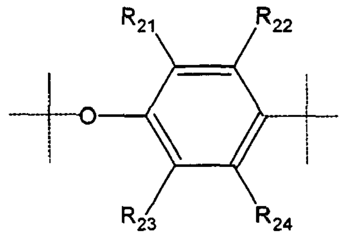

oder

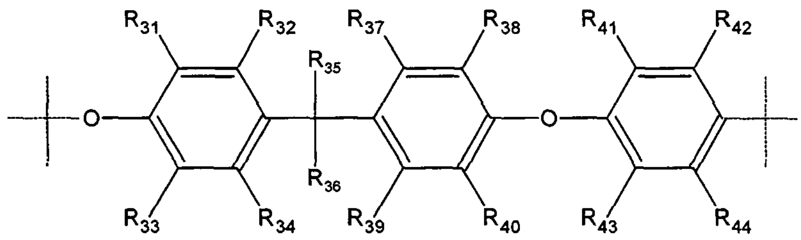

steht, und
$T_2$ für

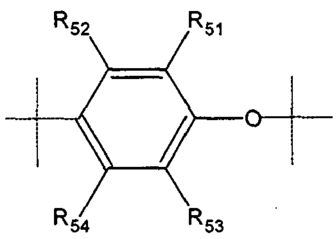

oder

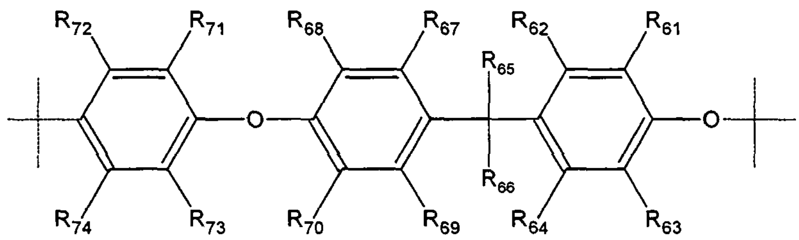

steht,
wobei $R_{21}$ bis $R_{74}$
jeweils unabhängig voneinander für Wasserstoff oder ein Halogenatom oder ein Pseudohalogen oder eine Hydroxygruppe oder eine Amingruppe oder eine Nitrogruppe oder Alkylgruppe mit 1 bis 20 Kohlenstoffatomen oder eine Cycloalkylgruppe mit 1 bis 20 Kohlenstoffatomen oder eine Alkenylgruppe mit 2 bis 20 Kohlenstoffatomen oder eine Alkinylgruppe mit 2 bis 20 Kohlenstoffatomen oder eine Arylgruppe oder eine Alkoxygruppe mit 1 bis 20 Kohlenstoffatomen oder eine Acylgruppe mit 1 bis 20 Kohlenstoffatomen oder eine Aldehydgruppe oder eine Carbonsäuregruppe oder eine Carbonsäureestergruppe mit 1 bis 20 Kohlenstoffatomen oder eine Amidgruppe oder eine Acylchlorid-, Acylbromid-, Acyliodid- oder Acylfluorid-Gruppe, stehen.

4. Zusammensetzung nach Anspruch 1, **dadurch gekennzeichnet, dass**

$R_1$, $R_2$ jeweils unabhängig voneinander für eine Nitrogruppe oder eine Trifluormethylgruppe,
$R_3$, $R_4$, $R_5$, $R_{13}$, $R_{14}$, $R_{15}$ für Wasserstoff,
$R_6$, $R_{16}$ für eine Phenylgruppe und
m, n jeweils unabhängig voneinander für 0 oder 1

stehen, wobei

$R_{21}$ bis $R_{34}$und $R_{37}$ bis $R_{44}$ für Wasserstoff und
$R_{35}$, $R_{36}$ jeweils unabhängig voneinander für eine Nitrogruppe oder eine Trifluormethylgruppe

stehen.

**5.** Zusammensetzung nach Anspruch 1, **dadurch gekennzeichnet, dass** der Anteil der Zusammensetzung an Verbindungen der allgemeinen Formel (I) $\geq$ 0,01 Gew.-% bis $\leq$ 1 Gew.-%, bezogen auf das Gesamtgewicht der Zusammensetzung, beträgt.

**6.** Verbindung der allgemeinen Formel (I)

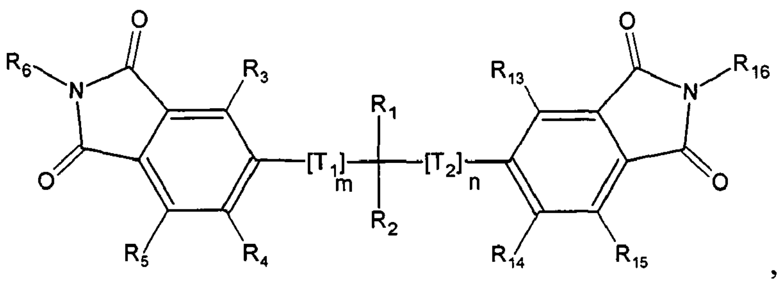
,

wobei

m, n jeweils unabhängig voneinander für eine Zahl $\geq$ 1 bis $\leq$ 10 stehen, und

$T_1$ für

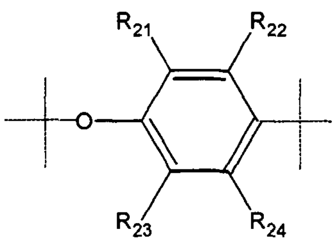

oder

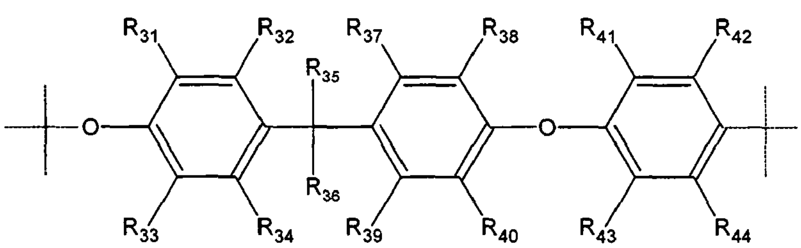

steht, und $T_2$ für

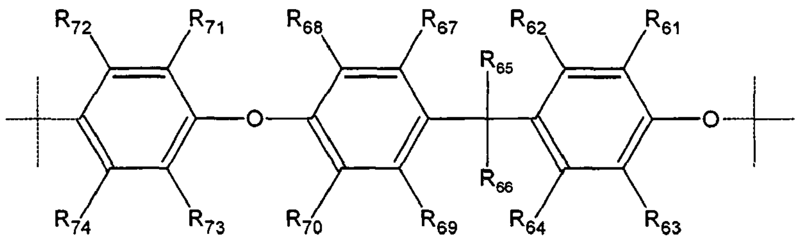

steht,
wobei $R_1$, $R_2$, $R_3$, $R_4$, $R_5$, $R_6$, $R_{13}$, $R_{14}$, $R_{15}$, $R_{16}$, $R_{21}$ bis $R_{74}$ jeweils unabhängig voneinander für Wasserstoff oder ein Halogenatom oder ein Pseudohalogen oder eine Hydroxygruppe oder eine Amingruppe oder eine Nitrogruppe oder Alkylgruppe mit 1 bis 20 Kohlenstoffatomen oder eine Cycloalkylgruppe mit 1 bis 20 Kohlenstoffatomen oder eine Alkenylgruppe mit 2 bis 20 Kohlenstoffatomen oder eine Alkinylgruppe mit 2 bis 20 Kohlenstoffatomen oder eine Arylgruppe oder eine Alkoxygruppe mit 1 bis 20 Kohlenstoffatomen oder eine Acylgruppe mit 1 bis 20 Kohlenstoffatomen oder eine Aldehydgruppe oder eine Carbonsäuregruppe oder eine Carbonsäureestergruppe mit 1 bis 20 Kohlenstoffatomen oder eine Amidgruppe oder eine Acylchlorid-, Acylbromid-, Acyliodid- oder Acylfluorid-Gruppe, stehen.

**7.** Polymerelement, umfassend eine Zusammensetzung nach Anspruch 1.

**8.** Verfahren zur Herstellung eines Polymerelements nach Anspruch 7 in dem das Polymerelement aus einer Zusammensetzung nach Anspruch 1 durch Pressen, Rakeln, Streichen, Schleudern, Sprühen, Extrusion, Blasformen und/oder Kalandern hergestellt wird.

**9.** Verfahren nach Anspruch 8, **dadurch gekennzeichnet, dass** das Polymerelement für ≥ 5 min bis ≤ 24 h bei ≥ 90 °C bis ≤ 200 °C getempert wird.

**10.** Elektretelement, umfassend ein aufgeladenes Polymerelement nach Anspruch 7.

**11.** Verfahren zur Herstellung eines Elektretelements nach Anspruch 10, in dem ein Polymerelement aus einer Zusammensetzung nach Anspruch 1 durch Pressen, Rakeln, Streichen, Schleudern, Sprühen, Extrusion, Blasformen und/oder Kalandern hergestellt und aufgeladen wird.

**12.** Verfahren nach Anspruch 11, **dadurch gekennzeichnet, dass** das Aufladen durch Coronaentladung erfolgt.

**13.** Verfahren nach Anspruch 11, **dadurch gekennzeichnet, dass** das Polymerelement vor dem Aufladen für ≥ 5 min bis ≤ 24 h bei ≥ 90 °C bis ≤ 200 °C getempert wird.

**14.** Elektromechanischer Wandler, umfassend ein Polymerelement nach Anspruch 7 oder ein Elektretelement nach Anspruch 10 oder ein Polymerelement, hergestellt durch das Verfahren nach Anspruch 8, oder ein Elektretelement, hergestellt durch das Verfahren nach Anspruch 11.

**15.** Verwendung einer Verbindung der allgemeinen Formel (I):

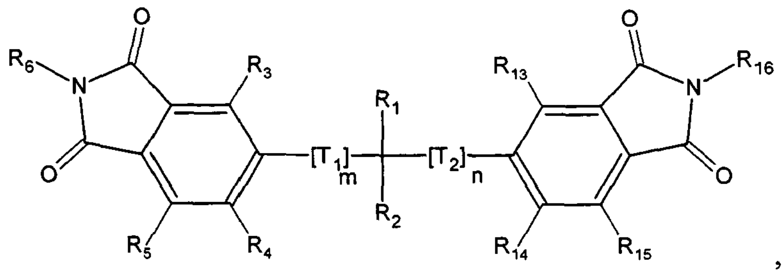

,

wobei

$R_1$, $R_2$, $R_3$, $R_4$, $R_5$, $R_6$, $R_{13}$, $R_{14}$, $R_{15}$, $R_{16}$ jeweils unabhängig voneinander für Wasserstoff oder ein Halogenatom oder ein Pseudohalogen oder eine Hydroxygruppe oder eine Amingruppe oder eine Nitrogruppe oder Alkylgruppe mit 1 bis 20 Kohlenstoffatomen oder eine Cycloalkylgruppe mit 1 bis 20 Kohlenstoffatomen oder eine Alkenylgruppe mit 2 bis 20 Kohlenstoffatomen oder eine Alkinylgruppe mit 2 bis 20 Kohlenstoffatomen oder eine Arylgruppe oder eine Alkoxygruppe mit 1 bis 20 Kohlenstoffatomen oder eine Acylgruppe mit 1 bis 20 Kohlenstoffatomen oder eine Aldehydgruppe oder eine Carbonsäuregruppe oder eine Carbonsäureestergruppe mit 1 bis 20 Kohlenstoffatomen oder eine Amidgruppe oder eine Acylchlorid-, Acylbromid-, Acyliodid- oder Acylfluorid-Gruppe, stehen, und
m, n jeweils unabhängig voneinander für eine Zahl $\geq 0$ bis $\leq 10$ stehen, und
$T_1$, $T_2$ jeweils unabgängig voneinander für eine organische Gruppe stehen,

als Additiv zur Verbesserung der Elektreteigenschaften eines Polycarbonats.

**16.** Verwendung eines Polymerelements nach Anspruch 7 oder eines Elektretelements nach Anspruch 10 oder eines Polymerelements, hergestellt durch das Verfahren nach Anspruch 8, oder eines Elektretelements, hergestellt durch das Verfahren nach Anspruch 11, in einem Sensor, Aktuator und/oder Generator, beispielsweise in einem Mikrofon, Lautsprecher, Schwingungswandler, pyroelektrischen Detektor oder Kondensator, und/oder als selbsthaftender Film.

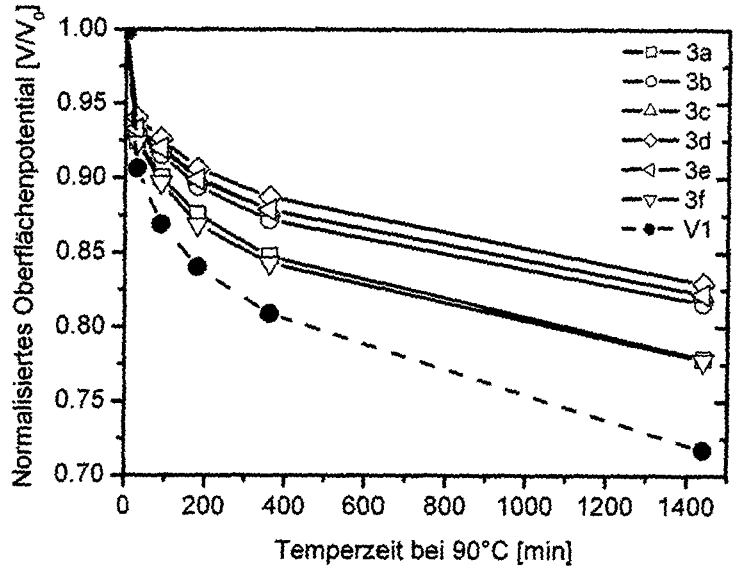
Normalisiertes Oberflächenpotential [V/V$_0$]
1.00
0.95
0.90
0.85
0.80
0.75
0.70
0
200
400
600
800
1000
1200
1400
Temperzeit bei 90°C [min]
3a
3b
3c
3d
3e
3f
V1

Fig. 1

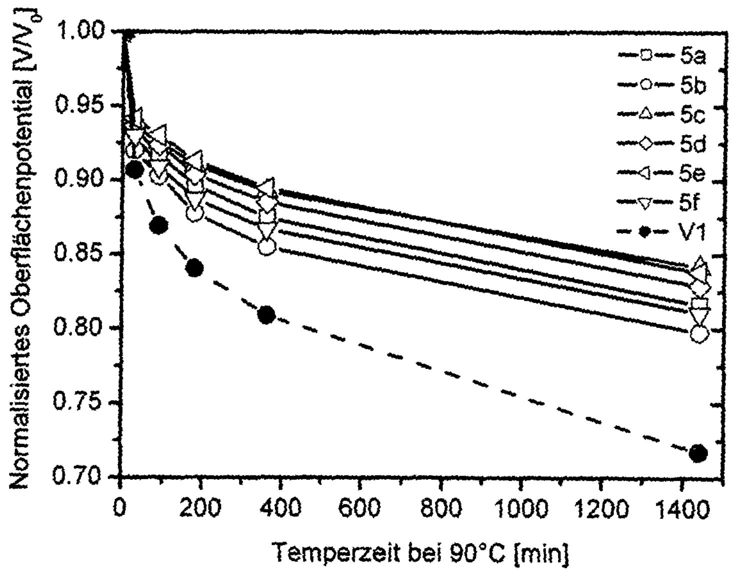
Normalisiertes Oberflächenpotential [V/V$_0$]
1.00
0.95
0.90
0.85
0.80
0.75
0.70
0
200
400
600
800
1000
1200
1400
Temperzeit bei 90°C [min]
5a
5b
5c
5d
5e
5f
V1

Fig. 2

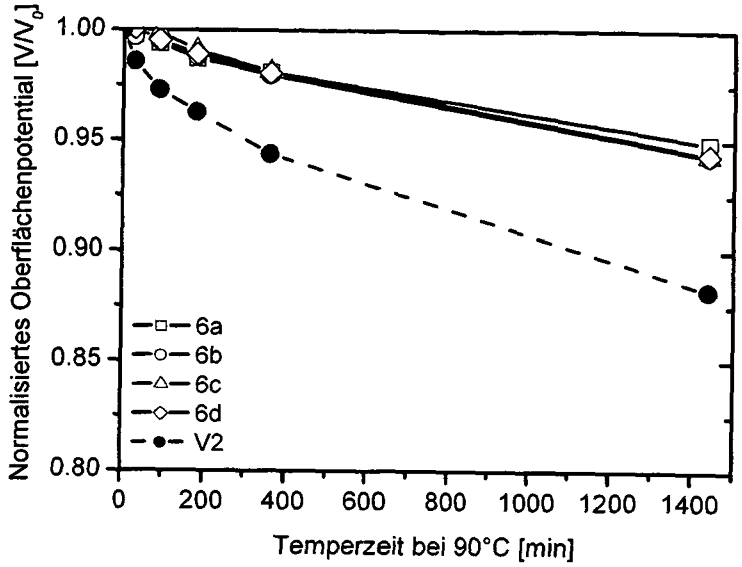
Normalisiertes Oberflächenpotential [V/V0]
1.00
0.95
0.90
0.85
0.80
0
200
400
600
800
1000
1200
1400
Temperzeit bei 90°C [min]
6a
6b
6c
6d
V2

Fig. 3

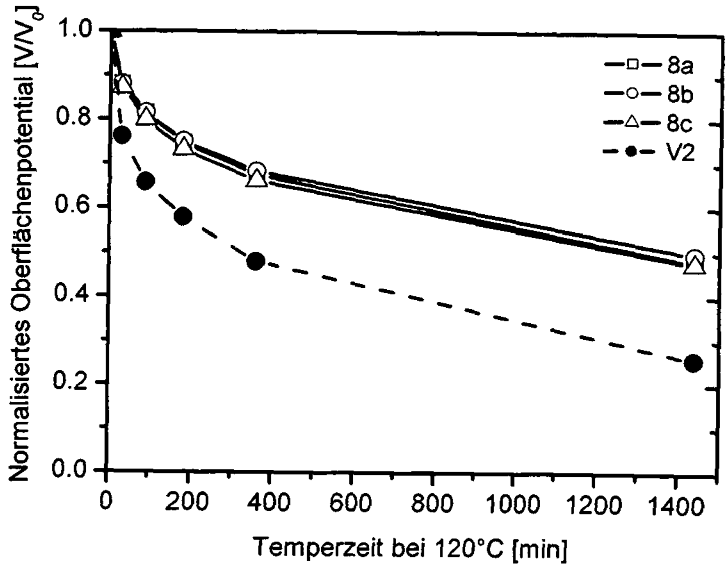
Normalisiertes Oberflächenpotential [V/V0]
1.0
0.8
0.6
0.4
0.2
0.0
0
200
400
600
800
1000
1200
1400
Temperzeit bei 120°C [min]
8a
8b
8c
V2

Fig. 4

Europäisches Patentamt
European Patent Office
Office européen des brevets

**EUROPÄISCHER RECHERCHENBERICHT**

Nummer der Anmeldung
EP 08 01 5097

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 5 976 208 A (ROUSSEAU ALAN D [US] ET AL) 2. November 1999 (1999-11-02)<br>* Ansprüche 1,2 *<br>* Spalte 6, Zeile 16; Beispiel 18 *<br>* Spalte 7, Zeilen 15-25 *<br>----- | 1-5, 7-11,15 | INV.<br>C07D209/02<br>C08K5/00<br>C08K5/3417<br>H04R19/01 |
| A | DATABASE REGISTRY<br>16. November 1984 (1984-11-16), "CAS RN 54395-52-7"<br>XP002513774<br>* das ganze Dokument *<br>----- | 6 | |
| A | DATABASE REGISTRY<br>16. November 1984 (1984-11-16), "CAS RN 74938-85-5"<br>XP002513775<br>* das ganze Dokument *<br>----- | 6 | |
| A | US 2007/003081 A1 (RAM SUNDER [US] ET AL) 4. Januar 2007 (2007-01-04)<br>* Absatz [0035]; Ansprüche 1,7 *<br>----- | 1-16 | RECHERCHIERTE SACHGEBIETE (IPC) |
| A | US 3 612 778 A (MURPHY PRESTON V) 12. Oktober 1971 (1971-10-12)<br>* Spalte 2, Zeilen 43-46; Anspruch 1 *<br>* Spalte 3, Zeilen 26-51 *<br>* Spalte 5, Zeilen 20-48 *<br>----- | 1-16 | C07D<br>C08K<br>H04R<br>B01D<br>D04H<br>D01F<br>C08L |

Der vorliegende Recherchenbericht wurde für alle Patentansprüche erstellt

| Recherchenort | Abschlußdatum der Recherche | Prüfer |
|---|---|---|
| München | 6. Februar 2009 | Dury, Olivier |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

2

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 08 01 5097

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

06-02-2009

| Im Recherchenbericht angeführtes Patentdokument | Datum der Veröffentlichung | Mitglied(er) der Patentfamilie | Datum der Veröffentlichung |
|---|---|---|---|
| US 5976208 A | 02-11-1999 | AU 700461 B2 | 07-01-1999 |
| | | AU 6648196 A | 12-03-1997 |
| | | BR 9610200 A | 04-08-1998 |
| | | CA 2227422 A1 | 27-02-1997 |
| | | CN 1192790 A | 09-09-1998 |
| | | DE 69627670 D1 | 28-05-2003 |
| | | DE 69627670 T2 | 08-04-2004 |
| | | EP 0845058 A1 | 03-06-1998 |
| | | ES 2195001 T3 | 01-12-2003 |
| | | JP 4069195 B2 | 02-04-2008 |
| | | JP 11510862 T | 21-09-1999 |
| | | WO 9707272 A1 | 27-02-1997 |
| | | US 5908598 A | 01-06-1999 |
| | | US 5968635 A | 19-10-1999 |
| | | US 5919847 A | 06-07-1999 |
| | | US 6002017 A | 14-12-1999 |
| US 2007003081 A1 | 04-01-2007 | KEINE | |
| US 3612778 A | 12-10-1971 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0845058 B1
- US 4196277 A
- US 5116939 A


### In der Beschreibung aufgeführte Nicht-Patentliteratur


- **Mohmeyer et al.** *Macromolecules,* 2006, vol. 39, 5760-5767